# FASCICULE DE BREVET EUROPEEN

(11) **EP 3 019 501 B1**
(45) Date de publication et mention de la délivrance du brevet: **08.08.2018**
(21) Numéro de dépôt: 14749914.9
(22) Date de dépôt: 08.07.2014
(51) Int. Cl.: C07D 493/04, C07D 519/00, C09D 4/00, C08G 63/06, C08L 67/07

(54) **UTILISATION POUR LA FABRICATION DE REVETEMENTS POLYMERES DE NOUVEAUX COMPOSES DERIVES DE 1:4, 3:6 DIANHYDROHEXITOL**
VERWENDUNG NEUARTIGER VERBINDUNGEN AUS 1,4:3,6-DIANHYDROHEXITOL ZUR HERSTELLUNG VON POLYMERBESCHICHTUNGEN
USE OF NOVEL COMPOUNDS DERIVED FROM 1,4:3,6 DIANHYDROHEXITOL FOR THE PRODUCTION OF POLYMER COATINGS

(30) Priorité: 08.07.2013 FR 1356686
(43) Date de publication de la demande: 18.05.2016
(73) Titulaire: Roquette Frères, 62136 Lestrem (FR); Centre National de la Recherche Scientifique (C.N.R.S.), 75794 Paris Cedex 16 (FR); UNIVERSITE DE MONTPELLIER, 34090 Montpellier (FR); Ecole Nationale Superieure de Chimie, 34000 Montpellier (FR)
(72) Inventeur: IBERT, Mathias, F-59930 La Chapelle d'Armentieres (FR); BUFFE, Clothilde, F-59160 Lomme (FR); SAINT-LOUP, René, F-59160 Lomme (FR); FERTIER, Laurent, F-86000 Poitiers (FR); GIANI, Olivia, F-34920 Le Crès (FR); ROBIN, Jean-Jacques, F-34830 Clapiers (FR); JOLY-DUHAMEL, Christine, F-34150 Gignac (FR)
(74) Mandataire: Cabinet Plasseraud
(86) Numéro de dépôt international: PCT/FR2014/051750
(87) Numéro de publication internationale: WO 2015/004381

(56) Documents cités:
- EP-A1- 2 644 589
- DE-A1- 19 939 403
- US-A1- 2013 017 484
- DATABASE REGISTRY [Online] CHEMICAL ABSTRACTS SERVICE, COLUMBUS, OHIO, US; 18 mars 2003 (2003-03-18), XP002715991, Database accession no. 499777-08-1
- DATABASE REGISTRY [Online] CHEMICAL ABSTRACTS SERVICE, COLUMBUS, OHIO, US; 25 juillet 2012 (2012-07-25), XP002715992, Database accession no. 1384259-33-9
- DATABASE REGISTRY [Online] CHEMICAL ABSTRACTS SERVICE, COLUMBUS, OHIO, US; 25 juillet 2012 (2012-07-25), XP002715993, Database accession no. 1384258-92-7
- HEISS ET AL.: "Development of a bioresorbable self-hardening bone adhesive based on a composite consisting of polylactide methacrylates and beta-tricalcium phosphate", JOURNAL OF BIOMEDICAL MATERIALS RESEARCH, PART B: APPLIED BIOMATERIALS, vol. 90b, no. 1, 2009, pages 55-66, XP002716004,
- BOXBERG ET AL.: "Effect of hydrophilicity on the properties of a degradable polylactide", JOURNAL OF POLYMER SCIENCE, PART B: POLYMER PHYSICS, vol. 44, no. 4, 2006, pages 656-664, XP002716005,

## Description

### Domaine de l'invention

L'invention a pour objet de nouveaux composés dérivés de 1:4, 3:6 dianhydrohexitol utiles pour la fabrication de polymères. Plus particulièrement l'invention concerne l'utilisation de ces composés dérivés de 1:4, 3:6 dianhydrohexitol pour la fabrication de revêtement polymère.

### Etat de la technique

De nombreuses industries ont besoin de compositions permettant de fabriquer des polymères, par exemple sous forme de revêtements polymères. Il peut s'agir par exemple de revêtements protecteurs, décoratifs, ou de traitement de surface. Il existe déjà un grand nombre de compositions réticulables pour cet usage dans la littérature et dans le commerce. Ces compositions consistent pour la plupart en un mélange de monomères polymérisables fabriqués par l'industrie chimique à partir de dérivés du pétrole.

Cependant, dans le contexte actuel de la diminution progressive des ressources en produits pétroliers, il est de plus en plus intéressant de remplacer les produits d'origine pétrolière par des produits d'origine naturelle.

L'utilisation de polymères biosourcés, c'est-à-dire réalisés à partir de matières premières d'origine naturelle, a déjà été décrite. En particulier, la publication de L. Jasinska et de C. E. Koning (« Unsaturated, biobased polyesters and their cross-linking via radical copolymerization », Journal of Polymer Science Part A: Polymer Chemistry, Volume 48, Issue 13, pages 2885-2895, 1 Juillet 2010) décrit la préparation de polyesters insaturés ayant des températures de transition vitreuse (T_{g}) relativement élevées, i.e. supérieures à 45°C, ces polyesters étant particulièrement utiles à la fabrication de revêtements. Ces polyesters insaturés sont obtenus par polymérisation d'isosorbide avec de l'anhydride maléique et optionnellement de l'acide succinique. Pour pouvoir être utilisés en tant que revêtement, ces polyesters insaturés doivent cependant être réticulés. Les polymères peuvent soit être mis en solution ou en suspension avec un agent réticulant, puis appliqués sur le substrat, et enfin réticulés après évaporation du solvant, soit déposés par des techniques usuelles de type « powder coating », soit fondus pour être déposés. Dans tous les cas, ces techniques de dépôt nécessitent une étape de post-réticulation. Le fait que la réalisation du revêtement nécessite plusieurs étapes successives est contraignant.

Le document Journal of Macromolecular Science, Part A : Pure and Applied Chemistry, 2006, Vol 43, Pages 967-975 décrit la fabrication de copolyesters fabriqués à partir d'ε-caprolactone et de poly(isosorbide-co-acide subérique).

Le document Macromolecular Symposia, 2009, Vol 283-284, Pages 144-151 décrit la fabrication de polyester à partir d'isosorbide et d'ε-caprolactone.

Ces deux derniers documents ne décrivent pas la fabrication de revêtements à partir des compositions décrites.

D'autres compositions réticulables, mais non utilisées pour la fabrication de revêtements, ont déjà été décrites.

Ainsi, le document DE 19939403 A1 décrit une famille de composés, utiles à la fabrication de pièces composites destinées à être utilisés en médecine humaine et vétérinaire, par exemple pour la fabrication d'implants. Ces composés ne sont pas utilisés pour la fabrication de revêtements. Les composés exemplifiés sont tous fabriqués à partir de lactide.

Le document US 20130017484 A1 vise quant à lui des composés utiles à la fabrication de polymères, qui comprennent une unique fonction réactive, cette fonction pouvant être une fonction acrylate ou méthacrylate. Aucun composé ne comprenant deux fonctions (méth)acrylates n'est décrit dans ce document.

Le document Development of a Bioresorbable Self-Hardening Bone Adhesive Based on a Composite Consisting of Polylactide Methacrylates and β-Tricalcium Phosphate, Heiss et al., Journal of Biomedical Materials Research, Part B: Applied Biomaterials, Vol.90b, n°1, 2009, pages 55-66, ainsi que le document Effect of Hydrophilicity on the Properties of a Degradable Polylactide, Boxberg et al., Journal of Polymer Science, Part B: Polymer Physics, Vol.44, n°4, 2006, pages 656-664, divulguent des résines utiles comme biomatériaux, comprenant des composés méthacrylés fabriqués à partir de lactide et d'isosorbide, ces composés étant ensuite réticulés.

La demande de brevet WO 2011/048739 décrit des compositions comprenant des composés (méth)acrylates cycliques ayant une formule générale spécifique, lesdits composés pouvant être obtenus par (méth)acrylation d'isosorbide. Ces composés constituent des monomères susceptibles de polymériser entre eux par voie radicalaire. On parle alors d'homopolymérisation. L'avantage d'employer de tels dérivés (méth)acryliques est de permettre une réticulation rapide, par voie thermique ou photochimique. Cependant les revêtements obtenus à partir de ces compositions par homopolymérisation présentent une résistance au choc assez faible et sont très cassants comme démontré ci-après.

Selon une variante de l'invention décrite dans ce document WO 2011/048739, les composés cycliques sont obtenus par modification préalable par de l'oxyde d'alkylène, avant d'être ensuite (méth)acrylés. Or, les oxydes d'alkylène sont sous forme gazeuse à température et pression ambiantes. La réaction de modification préalable est donc difficile à réaliser, notamment dans des installations industrielles. De plus, cette réaction préalable avec de l'oxyde d'alkylène peut poser des problèmes de sécurité. Aucun avantage de cette variante particulière n'est avancé dans cette demande et aucun revêtement à partir de ces composés n'a été réalisé et caractérisé.

Ce document WO 2011/048739 vise selon une autre variante des compositions à base de dérivés (méth)acrylates d'isosorbide et de dérivés (méth)acrylates de polyuréthane. Or, l'emploi de polymères porteurs de fonctions de type uréthane peut se révéler problématique en raison de l'instabilité thermique de la liaison uréthane (thermiquement réversible à des températures de l'ordre de 180-200°C), du dégagement de produits toxiques en cas de feu et de la toxicité des réactifs diisocyanates employés. De plus, il apparaît de cette demande WO 2011/048739 que l'ajout de polymères tels que les polyuréthanes porteurs de fonctions (méth)acrylates dans des compositions (méth)acrylates d'isosorbide diminue la résistance à l'abrasion du polymère obtenu à partir de cette composition. De plus, les revêtements obtenus à partir de ces dérivés (méth)acrylates d'isosorbide présentent une résistance au choc assez faible et sont très cassants.

Il serait donc avantageux de disposer de nouvelles compositions réticulables, au moins partiellement biosourcées, permettant d'obtenir des revêtements de qualité au moins équivalente, voire supérieure, par rapport aux revêtements obtenus avec les compositions déjà connues.

Un des objectifs de la présente invention est aussi de proposer une composition réticulable permettant de fabriquer des revêtements de manière plus facile, de préférence sans étape de séchage ou ne nécessitant pas de fondre le produit à appliquer sur le support.

C'est pour répondre à cette attente que les inventeurs ont recherché de nouvelles compositions réticulables. Ils ont ainsi découvert que de nouveaux derivés (méth)acrylés particuliers de dianhydrohexitols permettaient de répondre à ces attentes.

### Résumé de l'invention

Ainsi, l'invention a pour objet, l'utilisation pour la fabrication de revêtement polymère, d'un composé (A) de formule : 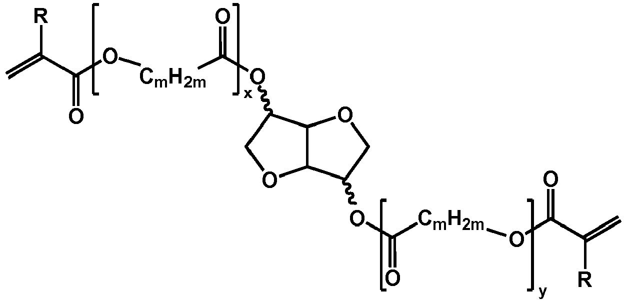 caractérisé en ce que les groupements R, identiques ou différents, sont choisis parmi les groupes H et CH₃ ;
- x est un entier allant de 0 à 100, avantageusement allant de 0 à 49, préférentiellement allant de 0 à 14, par exemple de 1 à 14 ;
- y est un entier allant de 0 à 100, avantageusement allant de 0 à 49, préférentiellement allant de 0 à 14, par exemple de 1 à 14 ;
- x+y est un entier supérieur ou égal à 1, avantageusement allant de 1 à 50, préférentiellement allant de 2 à 15, voire de 2 à 8 ;
- m est un entier égal à 5,
avec un amorceur de polymérisation et au moins un monomère additionel (B) susceptible de réagir avec ledit composé (A) et différent dudit composé (A). Ce composé permet de former des compositions réticulables, également utiles pour la formation de revêtements présentant de bonnes résistances au choc et à la déformation.

Le détail de l'invention va maintenant être développé dans la suite de la description.

### Description détaillée de l'invention

Le composé (A) décrit ci-dessus peut prendre différentes formes et être obtenu par les procédés décrits ci-après.

En ce qui concerne les groupes CₘH₂ₘ du composé (A), ce sont des groupes alkylènes à 5 atomes de carbone qui peuvent être linéaires ou ramifiés, préférentiellement linéaires.

Le composé (A) peut être obtenu par l'intermédiaire d'un composé (A') de formule :

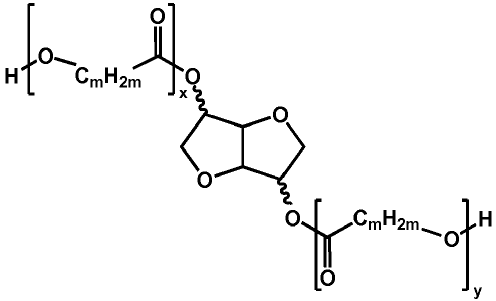

Où x, y et m ont les mêmes définitions que celles décrites précédemment pour le composé (A).

Ce composé (A') peut être obtenu par un procédé comprenant une étape de polymérisation en faisant réagir un 1:4, 3:6 dianhydrohexitol avec au moins un réactif, dit « réactif de polymérisation », qui est un composé choisi parmi les lactones, les hydroxyacides ou un mélange de ces composés.

Le 1:4, 3:6 dianhydrohexitol peut être choisi parmi l'isosorbide, isomannide et l'isoidide, et est préférentiellement l'isosorbide.

Le procédé de production de (A') comprend généralement une étape préalable d'addition dans un réacteur du 1:4, 3:6 dianhydrohexitol et du réactif de polymérisation, c'est-à-dire de lactone et/ou d'hydroxyacide. Ces réactifs peuvent être introduits séparément ou en mélange. De préférence, on utilise de 0,1 à 100 moles de réactif de polymérisation par mole de 1:4, 3:6 dianhydrohexitol, avantageusement de 0,5 à 15 moles, de préférence de 1 à 5 moles, par exemple de 1,5 à 4,5.

Le composé (A') peut ainsi être obtenu selon une première variante en faisant, lors de l'étape de polymérisation, réagir une lactone avec un 1:4, 3:6 dianhydrohexitol selon le schéma réactionnel 1 suivant :

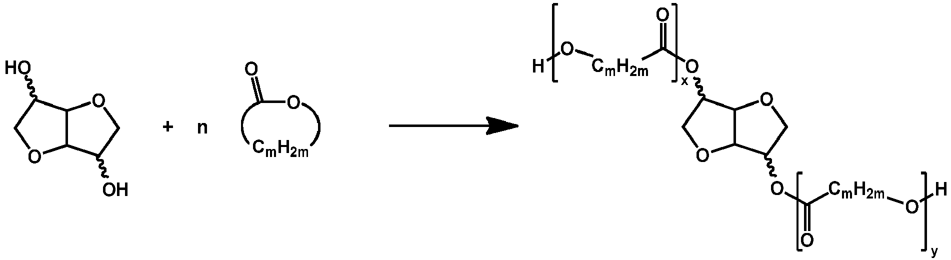

La lactone est avantageusement l'ε-caprolactone. Le choix de la lactone détermine, pour les composés (A) et (A'), si ces groupements sont linéaires ou ramifiés. Par exemple, lorsque l'on choisit l'ε-caprolactone, le groupement alkylène CₘH₂ₘ du composé (A'), et donc de (A), présente 5 atomes de carbone et est linéaire. Dans le cas où un mélange de lactones est utilisé, il est possible d'obtenir des composés (A') et par conséquent des composés (A) présentant des motifs alkylène qui diffèrent les uns des autres.

Le composé (A') peut également être obtenu, selon une seconde variante, en faisant, lors de l'étape de polymérisation, réagir un hydroxyacide avec un 1:4, 3:6 dianhydrohexitol selon le schéma réactionnel 2 suivant :

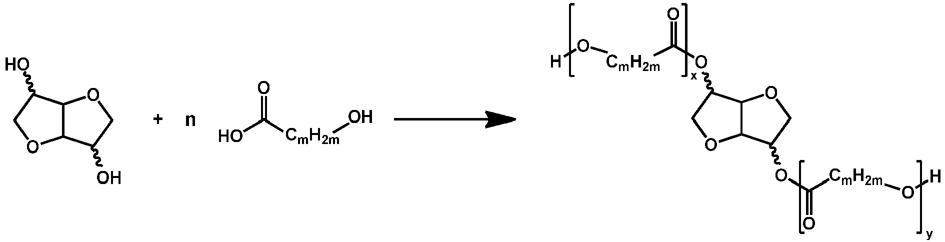

Avantageusement, l'hydroxyacide est choisi parmi l'acide hydroxycaproïque, l'acide 2-hydroxy-3,3-diméthylbutyrique, ou l'acide 2-hydroxyisocaproique, avantageusement l'acide hydroxycaproïque. Le choix de l'hydroxyacide détermine, pour les composés (A) et (A'), le nombre de carbones des groupements alkylène et si ces groupements sont linéaires ou ramifiés. Dans le cas où un mélange d'hydroxyacides est utilisé, il est possible d'obtenir des composés (A') et par conséquent des composés (A) présentant des motifs alkylène qui diffèrent les uns des autres.

Cette étape de polymérisation est généralement réalisée en présence d'un catalyseur afin de faciliter la réaction.

On peut par exemple utiliser un biocatalyseur tel que la lipase *Yarrowia lipolytica* comme décrit dans le document Macromolecular Symposia, 2009, Vol 283-284, Pages 144-151 où la lipase est immobilisée sur une résine support.

Il est également possible d'utiliser lors de l'étape de polymérisation un catalyseur chimique, par exemple du sodium métallique ou un catalyseur à base d'étain, comme décrit dans la partie « Exemples » ci-après. Il est possible d'utiliser entre 0,001 à 10% en masse de catalyseur par rapport à la masse totale des réactifs.

Cette réaction peut notamment se faire en phase solvant, par exemple dans le tétrahydrofurane, le toluène ou le diméthylformamide. La concentration en masse totale de réactifs dans le solvant, dépendante de la solubilité des réactifs dans ce solvant, est réglée classiquement et peut notamment aller de 10 à 2000 g/L, par exemple de 100 à 350 g/L.

Le milieu réactionnel peut être réglé à une température allant de 25 à 180 °C, par exemple de 60 à 120 °C, par exemple de 60 à 80 °C. La réaction peut éventuellement se faire à reflux.

Lorsque la réaction est réalisée en phase solvant, le solvant peut être éliminé à l'issue de la polymérisation, généralement par distillation qui peut être faite sous pression réduite.

Quel que soit le type de catalyseur éventuellement utilisé, la composition obtenue à l'issue de la polymérisation peut comprendre différents composés (A') et éventuellement des réactifs n'ayant pas réagi, par exemple du 1:4, 3:6 dianhydrohexitol et/ou des polymères de lactones ou d'hydroxyacides, ces polymères pouvant éventuellement être cyclisés.

Il est également possible de séparer les composés (A') des autres constituants par les techniques classiques de séparation. On peut par exemple effectuer à la fin de l'étape de polymérisation une ou plusieurs étapes de lavage de la composition obtenue, par exemple avec de l'eau, notamment avec de l'eau salée. On peut également utiliser d'autres techniques de séparation, par exemple les techniques de distillation, de chromatographie, de cristallisation ou de précipitation dans un ou plusieurs solvants.

On peut par exemple faire précipiter dans un solvant alcool la composition issue de la polymérisation, ledit solvant alcool étant avantageusement un alcool aliphatique comprenant de 1 à 6 atomes de carbone, préférentiellement de 1 à 4 atomes de carbone, par exemple dans le méthanol. Au contraire des autres constituants, les composés (A') précipitent dans le solvant alcool.

Il est également possible de faire précipiter dans un solvant éther la composition issue de la polymérisation, ledit solvant éther étant avantageusement un éther aliphatique comprenant de 1 à 6 atomes de carbone, préférentiellement de 1 à 4 atomes de carbone, par exemple l'éther éthylique. Les composés de haute masse moléculaire, notamment les composés de type polycaprolactone, précipitent et peuvent ainsi être séparées des composés (A') et des autres espèces de plus bas poids moléculaire.

Le procédé de fabrication comprend une étape de récupération du composé (A') ou de la composition comprenant (A') issue de la polymérisation. La composition obtenue comprend des composés (A') qui sont soit des composés de type (A'1) lorsque la valeur x ou y est égale à 0, soit des composés de type (A'2) lorsque la valeur de x et y est supérieure ou égale à 1. Autrement dit, la composition comprend des composés (A'1) dont une seule fonction OH du 1:4, 3:6 dianhydrohexitol a réagi avec le réactif de polymérisation et des composés (A'2) dont les deux fonctions OH du 1:4, 3:6 dianhydrohexitol ont réagi avec le réactif de polymérisation.

La composition obtenue à l'issue de la polymérisation peut ainsi comprendre des composés (A'1) et (A'2) selon un ratio molaire (A'2) / ((A'1)+(A'2)) pouvant aller de 0 à 1, préférentiellement allant de 0,05 à 0,8, notamment de 0,2 à 0,6. Le ratio molaire (A'2) / ((A'1)+(A'2)) peut être calculé par Résonnance Magnétique Nucléaire du Proton (RMN ¹H). A titre d'exemple, lorsque le 1:4, 3:6 dianhydrohexitol est de l'isosorbide, dans le diméthylsulfoxyde (DMSO), le pic caractéristique des composés (A'2) présente un déplacement chimique de 4,8 ppm, le pic caractéristique des composés de formule (A'1) dont la fonction hydroxyle en exo de l'isosorbide est substituée présente un déplacement chimique de 4,4 ppm et le pic caractéristique des composés de formule (A'1) dont la fonction hydroxyle en endo de l'isosorbide est substituée présente un déplacement chimique de 4,7 ppm ; le ratio molaire est alors calculé en faisant le rapport des intégrations des signaux RMN ¹H correspondant.

La composition issue de la polymérisation comprend généralement au moins 10% en masse, avantageusement au moins 40%, préférentiellement au moins 70%, tout préférentiellement au moins 90%, de composé (A').

Les quantités massiques de (A') peuvent être déterminées par analyse Chromatographie Phase Gazeuse, par exemple en utilisant le protocole décrit dans les exemples. Le pourcentage massique de (A') correspond alors au rapport de la somme de l'aire des pics correspondant aux espèces (A') sur la somme de l'aire de tous les pics.

Le composé (A) peut être obtenu par un procédé comprenant une étape de (méth)acrylation du composé (A') ou d'une composition comprenant au moins le composé (A'). Cette composition comprend généralement au moins 10% en masse de composé (A'), avantageusement au moins 40%, préférentiellement au moins 70%, tout préférentiellement au moins 90%. On obtient à l'issue de cette étape le composé (A) ou une composition comprenant le composé (A), appelée par la suite « composition de (méth)acrylation ».

Cette étape de (méth)acrylation peut se faire par exemple en faisant réagir le composé (A'), ou la composition le comprenant, avec un réactif de (méth)acrylation. Par exemple, elle peut se faire par réaction d'estérification des groupements hydroxyles du réactif (A') en utilisant comme réactif de (méth)acrylation un halogénure de (méth)acryloyle ou un anhydride d'acide (méth)acrylique. Elle peut également se faire par transestérification en employant un ester d'acide (meth)acrylique tel que le methacrylate de méthyle. Une autre possibilité est de réaliser une réaction de condensation d'acide (méth)acrylique avec les fonctions hydroxyles du réactif (A'), en éliminant l'eau à l'aide de composés carbodiimides ou par catalyse acide.

Préférentiellement, ce réactif est un halogénure de (méth)acryloyle, par exemple le chlorure de (méth)acryloyle. Dans ce dernier cas, la réaction se fait selon le schéma réactionnel 3 suivant :

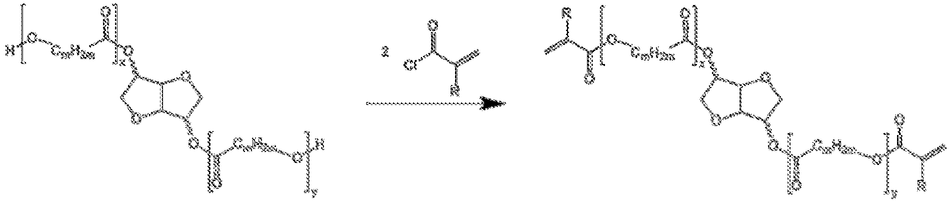

Selon la variante où un halogénure de (méth)acryloyle est utilisé pour réagir avec (A'), la réaction se fait généralement en présence d'une base, ladite base permettant la neutralisation de l'acide halohydrique formé lors de la réaction. Elle peut être choisie parmi les alkylamines, par exemple elle peut être la triéthylamine.

Du fait de la forte exothermicité de la réaction entre le réactif (A') et l'halogénure de (méth)acryloyle, cette étape de (méth)acrylation se fait généralement en refroidissant le milieu réactionnel, par exemple à une température allant de -10 à 10 °C.

La suite de la réaction peut être conduite à une température allant de 0 à 50 °C, par exemple à température ambiante. Elle peut être réalisée pendant une durée allant de 1 à 48 heures.

De préférence, cette réaction se fait en solvant. Ce solvant peut notamment être du dichlorométhane, du tétrahydrofurane, de l'acétone, du chloroforme ou de l'acétate d'éthyle. La concentration en masse totale de réactifs ((A') et réactif de (méth)acrylation) dans le solvant, dépendante de la solubilité des réactifs dans ce solvant, est réglée classiquement et peut notamment aller de 10 à 2000 g/L, par exemple de 100 à 1600 g/L.

Généralement, on introduit le réactif (A') avec la base dans le réacteur, et l'halogénure de (méth)acryloyle est introduit en goutte à goutte dans le réacteur sous agitation.

Comme expliqué précédemment, la composition soumise à l'étape de (méth)acrylation peut comprendre, en plus du composé (A'), d'autres constituants. Or, ces autres constituants, notamment les constituants comprenant des fonctions alcool, peuvent réagir avec le réactif de (méth)acrylation lors de l'étape de (méth)acrylation. Par exemple, lorsque la composition soumise à l'étape de (méth)acrylation comprend, en plus du composé (A'), des composés 1, 4: 3, 6 dianhydrohexitols et de la polycaprolactone linéaire, la composition de (méth)acrylation obtenue comprend au moins le composé (A) et des composés (méth)acrylés de 1, 4 : 3, 6 dianhydrohexitols et de polycaprolactone.

Le composé (A) peut être purifié par les méthodes classiques, notamment par distillation, chromatographie, cristallisation, précipitation dans un ou plusieurs solvants ou encore par extraction et lavage.

Le composé (A) présente avantageusement une faible température de transition vitreuse. Par faible température de transition vitreuse, on entend généralement une température de transition vitreuse inférieure à 20 °C, avantageusement inférieure à -20°C et préférentiellement inférieure à -35 °C.

Selon l'invention, on peut utiliser pour la fabrication de revêtement polymère une composition susceptible d'être obtenue par les procédés décrits précédemment. Selon l'invention, on peut également utiliser une composition comprenant différents composés (A), notamment des composés présentant des valeurs (x+y) différents les uns des autres. De préférence, cette composition comprend au moins 90% en masse de ce composé (A). Ces compositions peuvent être caractérisées par une masse moléculaire moyenne en nombre allant de 400 g/mol à 3000 g/mol, avantageusement de 450 g/mol à 2000 g/mol. Préférentiellement, la masse moléculaire moyenne en poids va de 420 g/mol à 10000 g/mol, tout préférentiellement de 500 à 4000 g/mol.

Un autre objet de l'invention porte sur l'utilisation pour la fabrication de revêtements polymères d'une composition comprenant au moins un composé (A), utile comme monomère, un amorceur de polymérisation et au moins un monomère additionel (B) susceptible de réagir avec ledit composé (A) et différent dudit composé (A). Selon une variante, cette composition (composition utile à l'invention) comprend, par rapport à sa masse totale, au moins 10% en masse de composé (A), notamment au moins 40%, par exemple au moins 70%, ou encore au moins 90%.

Les quantités massiques de (A) peuvent être déterminées par analyse Chromatographie Phase Gazeuse, par exemple selon le protocole décrit dans les exemples.

Dans la présente invention, le terme « amorceur de polymérisation » désigne tout agent chimique capable de déclencher la réaction de polymérisation des monomères présents dans la composition.

Préférentiellement, l'amorceur est un amorceur radicalaire. Cet amorceur peut être un photoamorceur ou un amorceur thermique. L'amorceur est de préférence un photoamorceur.

Les amorceurs thermiques préférés pour la présente invention peuvent être choisis dans le groupe constitué par les peroxydes organiques, tels que le peroxyde de benzoyle, le méthyl-cyclo-hexanone peroxyde, le cumènehydroperoxyde, le diisopropylbenzène peroxyde, le di-*t-*butyl peroxyde, le peroxybenzoate de *t*-butyle, le peroxycarbonate de diisopropyle et le monocarbonate de *t*-butylperoxyisopropyle, et par les composés diazoïque, tels que le 2,2'-azobis-isobutyronitrile (AIBN).

Les photoamorceurs préférés pour la présente invention peuvent être choisis dans le groupe constitué par les cétones aromatiques, tels que la benzophénone, les composés aromatiques, tels que l'anthracène, l'a-chlorométhylnaphtalène et le diphénylsulfide, et les composés soufrés, tels que le thiocarbamate. En particulier, le photoamorceur peut être choisi parmi les photoamorceurs activés par irradiation UV, par exemple ceux du groupe constitué par: l'acétophénone, l'acétophénone benzylcétal, la 1-hydroxycyclohexyl-phénylcétone, la 2,2-diméthoxy-1,2-diphényléthane-1-one, la xanthone, la fluorènone, le benzaldéhyde fluorène, l'anthraquinone, la triphénylamine, le carbazole, la 3-méthyl-acétophénone, la 4-chloro-benzophénone, la 4,4'-méthoxy-benzophénone, la 4,4'-diamino-benzophénone, le benzoinpropyléther, le benzoinéthyl éther, la benzyl méthyl cétal 1-(4-isopropylphényl)-2-hydroxy-2-méthyl-propane-1-one, la 2-hydroxy-2-méthyl-1-phénylpropan-1-one, la thioxanthone, la diéthyl-thioxanthone, la 2-isopropyl-thioxanthone, la 2-chlorothioxanthone, la 2-méthyl-1-[4-(méthyl-thio)-phényl]-2-morpholinopropan-1-one, la 2-benzyl-2-diméthyl-amino-1-(4-morpholino-phényl)-butan-1,4-(2-hydroxyéthoxy)-phényl-(2-hydroxy-2-propyl) cétone, l'oxyde de 2,4,6-triméthylbenzoyl diphényl-phosphine, l'oxyde de bis-(2,6-méthoxybenzoyl)-2,4,4-triméthyl-pentyl phosphine, et l'oligo-(2-hydroxy-2-méthyl-1-(4-(1-méthylphényl)phényl)-propanone).

Des photoamorceurs de polymérisation disponibles dans le commerce sont, par exemple, les produits Irgacure® 184, 369, 651, 500, 819, 907, 784, 2959, CGI 1700, CGI 1750, CGI 1850 et CG24-61, Darocur® 1116 et 1173, et Lucirin® TPO de BASF, le produit Uvecryl® P36 d'UCB, et les produits Esacure® KIP 150, KIP 65LT, KIP 100F, KT37, KT55, KTO46 et KIP75/B de FratelliLamberti.

L'amorceur de polymérisation peut consister en un mélange de plusieurs amorceurs de polymérisation, par exemple en un mélange d'un amorceur thermique et d'un photoamorceur. La quantité massique d'amorceur pourra être adaptée par l'homme du métier. Cette quantité massique peut aller de 0,01 à 20 %, préférentiellement de 0,1 à 5 % de la masse totale des composés (A).

On peut également ajouter à la composition utile à l'invention, notamment lorsque l'amorceur est un amorceur thermique, un ou plusieurs catalyseurs permettant d'augmenter la vitesse de décomposition de l'amorceur ou encore de rendre efficace l'amorceur à plus basse température. Ces catalyseurs peuvent être de type amine.

La composition utile à l'invention comprend en outre, au moins un monomère additionnel (B), différent du composé (A) et susceptible de réagir avec ce dernier. Le revêtement obtenu à partir de cette composition préférée présente, et notamment dans ses variantes préférées, des caractéristiques de brillant ou un indice de brillance tout à fait satisfaisants.

Ces monomères additionnels (B) peuvent être par exemple les monomères compris dans la composition de (méth)acrylation, notamment des composés (méth)acrylates de 1, 4 : 3, 6 dianhydrohexitols et de polycaprolactone. Avantageusement, (B) est un (méth)acrylate de 1, 4 : 3, 6 dianhydrohexitol. Le composé (méth)acrylate de 1, 4 : 3, 6 dianhydrohexitol peut être, de préférence, choisi parmi le diacrylate d'isosorbide, le diméthacrylate d'isosorbide ou un mélange de diacrylate(s) d'isosorbide et de diméthacrylate(s) d'isosorbide.

Cette composition comprend préférentiellement, par rapport à la quantité totale de (A) et (B), de 1 à 99,99% en masse de (A) et de 0,01 à 99% en masse de (B). Avantageusement, la composition comprend de 20 à 85% en masse de (A) et de 15 à 80% en masse de (B). De préférence la composition comprend de 40 à 60 % en masse de (A) et de 40 à 60 % en masse de (B).

La composition utile à l'invention peut comprendre également comme monomère additionnel (B) des composés comprenant au moins une fonction (méth)acrylate, notamment des composés (méth)acrylate d'alkyle tel que le méthacrylate de méthyle. Il peut également s'agir d'un ou plusieurs monomères oligomériques fonctionnalisés porteurs d'au moins une fonction acrylate ou méthacrylate, ledit monomère oligomérique présentant des motifs répétitifs choisis parmi les polyuréthanes, les polyéthers ou les polyesters aliphatiques ou semi-aliphatiques de faible température de transition vitreuse telle que définie précedemment. Le polyéther peut notamment être un polyéthylène glycol (PEG), un polypropylène glycol (PPG), et polytétraméthylène glycol (PTMG). Le polyester aliphatique ou semi-aliphatique de faible température de transition vitreuse peut notamment être la poly ε-caprolactone ou la poly butyrolactone.

Le monomère oligomérique peut présenter une masse moléculaire moyenne en nombre allant de 300 à 5000 g.mol⁻¹, préférentiellement de 350 à 2000 g.mol⁻¹, tout préférentiellement de 400 à 1000 g.mol⁻¹.

Ce monomère (B) peut comprendre au moins une fonction promotrice d'adhésion. Dans ce dernier cas, on peut parler pour le monomère (B) de promoteur d'adhésion.

Une fonction promotrice d'adhésion est une fonction chimique susceptible d'établir des liaisons chimiques ou physiques fortes ou faibles avec la surface d'un substrat.

De préférence, la fonction promotrice d'adhésion peut être choisie parmi la fonction acide carboxylique, notamment les fonctions acrylique ou méthacrylique, les fonctions comprenant au moins un atome de phosphore sous forme acide ou ester, les fonctions éthoxy- ou méthoxy-silane ou les fonctions silanol.

Ce monomère promoteur d'adhésion peut être l'acide acrylique, l'acide méthacrylique, les composés insaturés dérivés d'acide phosphorique et d'ester d'acide phosphorique, les composés insaturés dérivés d'acide phosphonique et d'ester d'acide phosphonique, les composés insaturés alcoxysilanes, les composés insaturés porteurs de la fonction silanol.

Le monomère promoteur d'adhésion peut représenter de 0 à 15 %, par exemple de 1 à 15%, préférentiellement de 3 à 14 %, et encore plus préférentiellement de 7 à 12 % en poids de la composition selon l'invention.

La composition selon l'invention peut renfermer, outre le monomère (A) ou le mélange de monomères (A), un mélange des monomères (B) tels que décrits précédemment.

La composition peut également comprendre au moins un agent d'étalement ayant pour fonction de permettre, avant réticulation, un bon étalement de la composition lorsqu'elle est appliquée sur le substrat. L'agent d'étalement peut être particulièrement utile pour faciliter la formation de revêtements.

La composition peut également comprendre d'autres constituants non copolymérisables avec les monomères de la composition. Il peut s'agir par exemple d'inhibiteurs de polymérisation, de colorants ou de pigments, d'opacifiants ou d'additifs de protection thermique ou ultra-violet, d'agents anti-statiques, de polymères différents de (A) et (B), d'agents antibactériens, anti-salissures ou anti-fongiques. De préférence cependant, la composition selon l'invention comprend moins de 10%, plus préférentiellement moins de 2% en poids de ces autres constituants.

La composition selon l'invention peut être obtenue en mélangeant les monomères (A) ainsi que les éventuels monomères (B) et autres constituants non copolymérisables.

Comme indiqué précédemment, la composition selon l'invention peut comprendre des inhibiteurs de polymérisation.

Une composition décrite dans la présente invention comprend en masse :
- de 1 à 99,99 % monomère (A), avantageusement de 20 à 85%, de préférence de 40 à 60 % ;
- de 0 à 98,99 % monomère (B), avantageusement de 15 à 80%, de préférence de 40 à 60 % ;
- de 0,01 à 20 % d'amorceur ;
- de 0 à 10 % de constituant non copolymérisable ;
la somme des constituants faisant 100%.

Le revêtement obtenu à partir de cette composition préférée présente, et notamment dans ses variantes préférées, des caractéristiques de brillant ou un indice de brillance tout à fait satisfaisants.

La composition utile à l'invention se présente avantageusement sous forme liquide à température ambiante. La viscosité de la composition peut aller jusqu'à 15000 Poises à 25 °C. Le composé, (composé (A)) et la composition utiles à l'invention peuvent être utilisés pour la fabrication de polymères réticulés. Le polymère réticulé, notamment lorsqu'il est sous la forme de revêtement polymère réticulé, comme présenté ci-après, présente une température de transition vitreuse généralement inférieure à 20 °C, avantageusement inférieure à -20 °C et préférentiellement inférieure à -35 °C.

En particulier, le composé et la composition utiles à l'invention peuvent être utilisés pour la fabrication de revêtement polymère, ledit revêtement pouvant être appliqué sur tout type de substrat. Par « revêtement », on entend dans la présente invention tout type de couche de faible épaisseur appliquée sur un substrat, quelle que soit la fonction de cette couche. Il peut s'agir par exemple de revêtements protecteurs, décoratifs, ou de traitement de surface. Les films, les vernis et les peintures font partie des revêtements au sens de la présente invention. Le substrat sur lequel est appliqué le revêtement dans la présente invention peut être de toute sorte. Il peut s'agir en particulier de substrats en bois, en métal, en plastique, en verre ou en papier.

L'invention a également pour objet un procédé de fabrication d'un revêtement polymère sur un substrat comprenant les étapes suivantes :
- le dépôt sur le substrat d'une couche de la composition selon l'invention telle que décrite ci-avant, puis
- la réticulation de la composition.

Le dépôt de la composition peut être réalisé selon tout moyen connu de l'homme du métier, par exemple par trempage, par enduction centrifuge, par « barcoater », par « tape casting » ou par pulvérisation. L'épaisseur de la couche déposée est ajustée en fonction de l'épaisseur du revêtement que l'on souhaite obtenir. L'épaisseur de la couche déposée peut par exemple être comprise entre 100 nm et 2 mm, par exemple entre 200 et 800 nm. De préférence, la couche a une épaisseur uniforme, de manière à obtenir un revêtement final uniforme.

La réticulation de la composition se fait par activation de l'amorceur de polymérisation. Cette activation varie en fonction du type d'amorceur contenu dans la composition. La durée et les conditions de l'étape d'activation de l'amorceur de polymérisation dépendent essentiellement de la nature et de la concentration des monomères et de l'amorceur dans la composition, et l'homme du métier saura adapter ces paramètres selon le cas.

Selon un mode de réalisation préféré, l'amorceur de polymérisation est un photoamorceur, et l'activation de ce photoamorceur est obtenue en exposant la couche de composition déposée sur le substrat à une irradiation, en particulier à une irradiation UV. De préférence, l'irradiation est effectuée de façon égale sur l'ensemble de la couche de composition déposée sur le substrat.

L'activation de l'amorceur de polymérisation permet de déclencher la réaction de polymérisation des monomères (A) entre eux, ainsi qu'avec les éventuels monomères (B), décrits précédemment.

L'amorceur peut être déjà présent dans la composition ou ajouté juste avant le dépôt de la composition sur la surface à revêtir.

L'amorceur est de préférence ajouté juste avant le dépôt de la composition sur la surface à revêtir.

Des étapes additionnelles sont possibles comme un recuit du revêtement ou un flammage du support avant application de la composition, mais ces étapes ne sont pas obligatoires pour réaliser le procédé selon l'invention.

Ce procédé permet d'obtenir un revêtement polymère présentant des propriétés intéressantes. En particulier, les revêtements obtenus peuvent présenter au moins une des propriétés suivantes :
- une bonne transparence ;
- une brillance élevée ;
- une bonne adhésion au substrat ;
- une dureté élevée ;
- une bonne résistance à l'abrasion ou à l'usure ;
- une bonne résistance chimique, aux solvants ou une bonne tenue à l'eau, mais aussi aux bases et aux acides ;
- une bonne tenue à l'impact/au choc ;
- ainsi qu'une bonne résistance à la déformation.

Ainsi, les revêtements obtenus selon la présente invention possèdent des propriétés au moins aussi bonnes, si ce n'est meilleures que des revêtements actuellement disponibles, et présentent en outre l'avantage d'être obtenus aisément à partir de matériaux biosourcés.

L'invention sera mieux comprise à la lumière des exemples non limitatifs et purement illustratifs suivants.

### Exemples

### Monomère (A)

### Synthèse de (A') à partir d'isosorbide et d'ε-caprolactone

Dans un réacteur d'une capacité de 50 mL, chauffé par un bain d'huile de silicone et muni d'un système de contrôle de la température du bain, d'un système d'agitation à barreau aimanté surmonté d'un réfrigérant, sont introduits 1,5 g d'isosorbide (0,01 mol) dissous dans 15 mL de tétrahydrofurane, puis 2,4 g d'ε-caprolactone (2 équivalents molaires par rapport à l'isosorbide) et 0,04 g de 2-éthylhexanoate d'étain. Le mélange est chauffé à 70°C pendant 24 heures. L'avancement de la réaction est suivi par RMN ¹H (CDCl₃). Le tétrahydrofurane a été évaporé sous pression réduite, puis le brut réactionnel est lavé trois fois avec de l'eau distillée et une fois avec une solution d'eau salée saturée. La phase organique a été séchée avec du sulfate de magnésium anhydre et le solvant est évaporé sous pression réduite. Après précipitation dans le méthanol, 3,0 g de composition issue de la polymérisation est obtenue sous forme d'une cire jaunâtre, qui est un mélange contenant des composés de type (A'1) et de type (A'2).

D'après l'analyse chromatographique en phase gazeuse, la teneur massique résiduelle en isosorbide est de 0,2%, la teneur massique résiduelle en caprolactone est de 0,3%. et la teneur en monomère (A') est de 93.5%, le reste étant principalement constitué de polycaprolactone.

La chromatographie en phase gaz est conduite sur un appareil Bruker 450 équipé d'une colonne de polydiméthylsiloxane de type Zebron ZB-1 HT; 30 m × 0,32 mm × 0,25 µm (épaisseur de film) équipé d'un détecteur de type FID chauffé à 300°C. La colonne est chauffée de 100 à 350 °C à une vitesse de 7°C/min puis est maintenue à 350°C pendant 15 minutes.

Les masses moléculaires moyennes ont été déterminées par MS-MALDI-TOF (DHB + Na): Mn = 650 g.mol⁻¹ ; Mw = 710 g.mol⁻¹

La composition obtenue, diluée dans le diméthylsulfoxyde avant analyse, présente un spectre RMN 1H dont les pics à 4,4 ppm sont caractéristiques des composés de formule (A'1) dont la fonction hydroxyle en exo de l'isosorbide est substituée, le pic à 4,7 ppm est caractéristique des composés de formule (A'1) dont la fonction hydroxyle en endo de l'isosorbide est substituée, et le pic à 4,8 ppm est caractéristique des composés de formule (A'2) dont les 2 fonctions hydroxyles sont substituées. Le rapport des aires des pics donne un ratio molaire (A'2)/(A'1+A'2) égal à 0,36.

La synthèse est répétée afin de produire suffisamment de composés (A1) et (A2) pour fabriquer et caractériser les polymères ensuite fabriqués.

### Synthèse de (A) par acrylation de (A') :

20 g des composés (A') synthétisés précédemment sont dilués dans 150 mL de dichlorométhane. Le milieu est refroidi à 0°C et de la triéthylamine (4 équivalents molaires par rapport à (A')) est additionnée. Après 5 minutes d'agitation, le chlorure d'acryloyle (4 équivalents molaires par rapport à (A')), dilué dans 15 mL dichlorométhane, est ajouté en goutte à goutte pendant 30 minutes. Lorsque le chlorure est introduit, la réaction est laissée sous agitation pendant 24 heures à température ambiante. L'avancement de la réaction est contrôlé par RMN ¹H (CDCl₃). Le milieu réactionnel est lavé trois fois avec de grands volumes de saumure. La phase organique est séchée sur sulfate de magnésium anhydre et évaporée. Le brut obtenu est nettoyé sur charbon actif (éluant : dichlorométhane). Le produit est obtenu sous la forme d'une cire jaune (27 g ; rendement 96%).
MS-MALDI-TOF (DHB + Na): Mn = 770 g.mol⁻¹ ; Mw = 850 g.mol⁻¹

### Monomère (B)

### Synthèse du diacrylate d'isosorbide

Le diacrylate d'isosorbide est obtenu à partir d'isosorbide selon le protocole suivant :

Dans un réacteur d'une capacité de 250 mL, refroidi à 0°C par un bain de glace salée et muni d'un système d'agitation à barreau aimanté, est introduit 3 g d'isosorbide (0,21 mol) dissous dans du dichlorométhane (120 mL) puis 8,3 g de triéthylamine (4 équivalents molaires par rapport à l'isosorbide) et 7,4 g de chlorure d'acryloyle dilués dans du dichlorométhane (30 mL), ajouté goutte à goutte. Le mélange est agité pendant 24 heures à température ambiante. L'avancement de la réaction est suivi par RMN ¹H dans le Chloroforme deutéré (CDCl₃). Le milieu réactionnel est lavé trois fois avec de grands volumes d'eau salée. Après séchage de la phase organique avec du sulfate de magnésium anhydre, le solvant est évaporé sous pression réduite. Le brut est purifié par chromatographie sur gel de silice (éluant : dichlorométhane). 4,14 g de composition (solide jaune) comprenant les composés (A) sont obtenus (rendement : 79%).

### Synthèse de diacrylate lactate isosorbide

Dans un réacteur d'une capacité de 50 mL, chauffé par un bain d'huile de silicone et muni d'un système de contrôle de la température du bain, d'un système d'agitation à barreau aimanté surmonté d'un réfrigérant, sont introduits 1,5 g d'isosorbide (0,01 mol) dissous dans 15 mL de tétrahydrofurane, puis 1,5 g de L-lactide (1 équivalent molaire par rapport à l'isosorbide) et 0,04 g de 2-éthylhexanoate d'étain. Le mélange est chauffé à 70°C pendant 24 heures. L'avancement de la réaction est suivi par RMN 1H (CDCl3). Le tétrahydrofurane a été évaporé sous pression réduite, puis le brut réactionnel est lavé trois fois avec de l'eau distillée et une fois avec une solution d'eau salée saturée. La phase organique a été séchée avec du sulfate de magnésium anhydre et le solvant est évaporé sous pression réduite. Après précipitation dans le méthanol, 2,5 g de composition de lactate isosorbide est obtenue sous forme d'une cire blanchâtre.

La composition de lactate isosorbide précédemment obtenue est ensuite acrylée dans les mêmes conditions que celles utilisées pour la formation du monomère (A). Le produit est obtenu sous la forme d'une cire jaune.
Promoteur d'adhésion : Sartomer® SR9054, Acide Acrylique (Sartomer Europe)

### Autres constituants

Amorceur : Darocur® 1173 (BASF)
Agent d'étalement : Perenol® F40 (BASF)

### Préparation de compositions réticulables

Différentes compositions utiles à la présente invention (C1 à C3) et comparatives (C4) et (C5) ont été préparées en mélangeant les composés dans les proportions en grammes indiquées dans le tableau suivants :
Ces compositions ont ensuite été homogénéisées par agitation et bain ultra-son.

**Tableau 1 : Compositions**

| | C.1 | C.2 | C.3 | C.4 | C.5 |
|---|---|---|---|---|---|
| Monomère (A) : | 10,0 g | 10,0 g | 10,0 g | 0 g | 0 g |
| Monomère (B) : | | | | | |
| - diacrylate d'isosorbide | 0 g | 3,9 g | 11,7 g | 10,0 g | 0 g |
| - diacrylate de lactate isosorbide | 0 g | 0 g | 0 g | 0 g | 10,0 g |
| - Acide Acrylique | 1,0 g | 1,4 g | 2,2 g | 1,0 g | 1,0 g |
| Amorceur | 0,05 g | 0,10 g | 0,20 g | 0,26 g | 0,07 g |
| Agent d'étalement | 1,0 g | 1,4 g | 2,2 g | 1,0 g | 1,0 g |

### Préparation du matériau :

Chacune des compositions réticulables telles que décrites ci-dessus ont été déposées dans des coupelles en aluminium de 4 cm de diamètre.

Les coupelles ont ensuite été posées sur le tapis roulant d'un banc UV (Fusion UV System, Inc®, modèle LC6E - Lampe à vapeur de mercure de type H (FUSION F300S)- 200nm-600nm - 120 W.cm⁻² - quelques secondes d'expositions) afin de procéder à la réticulation.

Pour chacune des compositions, 5 passages à 15 cm/s sous la lampe UV ont été réalisés. Des matériaux solides ont ainsi été obtenus et caractérisés thermiquement selon les protocoles suivants.

La température de transition vitreuse (Tg) exprimée en degré Celsius (°C) est mesurée par calorimétrie différentielle à balayage (2 passages de -100°C à 200°C, 10°C.min⁻¹). La température de dégradation à 10% de perte de masse (Td₁₀) exprimée °C a été mesurée par analyse thermogravimétrique (25°C à 600°C, 10°C.min⁻¹ sous air). Ces températures caractéristiques des matériaux formés sont reportées dans le Tableau 2 ci-dessous.

L'aspect cassant ou non du matériau à température ambiante est également reporté dans le Tableau.

**Tableau 2 : Caractérisations thermiques et mécaniques des matériaux obtenus**

| Composition | Tg (°C) | Aspect matériau | Td₁₀ (°C) |
|---|---|---|---|
| C.1 | -29 | NC | 280 |
| C.2 | -13 | NC | 300 |
| C.3 | -44 | NC | 310 |
| C.4 | 36 | C | 330 |
| C.5 | 75 | C | NM |

| | | | |
|---|---|---|---|
| NC : Non cassant // C : cassant | | | |

Les matériaux obtenus à partir des compositions utiles à la présente l'invention (C1, C2 et C3) ne sont pas cassants à température ambiante, contrairement aux matériaux de l'art antérieur, obtenus à partir d'acrylate d'isosorbide (C4) ou à partir de diacrylate lactate isosorbide (C5). Ces matériaux présentent en outre une excellente tenue thermique.

### Préparation du revêtement :

Une fine couche de chacune des compositions réticulables telles que décrites au Tableau 1 a été déposée sur des supports en acier (lame Q-Lab de type Q-panel acier laminé 76x152 mm) à l'aide d'un bar-coater Sheen Instruments 1133N, équipé d'une barre de 20 µm afin de recouvrir toute la surface du support avec le minimum de composition.

Les supports ainsi recouverts ont ensuite été déposés sur le tapis roulant d'un banc UV (Fusion UV System, Inc®, modèle LC6E - Lampe à vapeur de mercure de type H (FUSION F300S)- 200nm-600nm - 120 W.cm⁻² - quelques secondes d'expositions) afin de procéder à la réticulation.

Pour chacune des compositions, 5 passages à 15 cm/s sous la lampe UV ont été réalisés. Des revêtements d'une épaisseur de 20 µm ont ainsi été obtenus.

Les tests d'adhésion ont été effectués selon la norme ASTM D3359-09 « Standard test methods for measuring adhesion by tape test » sur une surface acier de type Q-panel et sur une lame de bois.

Les tests de résistance à la déformation et au choc ont été effectués selon la norme ASTM D2794-10 « Standard test method for resistance of organic coatings to the effects of rapid deformation (impact) ». Ces résistances au choc et à la déformation sont quantifiées par la puissance maximale d'impact en pourcentage. 100% représente un poids de 800g lâché d'une hauteur de 50 centimètres.

Les indices de brillance ont été mesurées selon la norme ASTM D523-08 « Standard Test Method for Specular Gloss » à un angle de 60° sur une lame d'acier.

**Tableau 3 : Caractérisations des revêtements (épaisseur ≈ 20µm)**

| Composition | Adhésion | | Résistance à la déformation | Résistance au choc | Indice de Brillance |
|---|---|---|---|---|---|
| | Acier | Bois | | | |
| C.1 | 4 | 5 | 100% | 100% | 25 |
| C.2 | 4 | 5 | 100% | 100% | 65 |
| C.3 | 5 | 5 | 80% | 100% | 90 |
| C.4 | 4 | NM | < 10% | 60% | NM |
| C.5 | 3 | NM | <10 % | 40% | NM |

Les revêtements selon la présente invention présentent une aussi bonne, voire meilleure adhésion sur tous les types de supports testés.

Les revêtements selon l'invention (C1, C2 et C3) présentent en outre l'avantage d'être très résistants au choc et à la déformation, au contraire des revêtements à base d'acrylate d'isosorbide de l'art antérieur (C4) ou ceux à base de diacrylate lactate isosorbide (C5).

Par ailleurs, si la composition utile à la présente invention combine la présence de monomère (A) et l'acrylate d'isosorbide comme monomère additionnel (B) (compositions C2 et C3), les résistances à la déformation et au choc restent maintenues. Ceci est d'autant plus surprenant que ces quantités peuvent dépasser 60% en masse d'acrylate d'isosorbide. En plus de comprendre des quantités en isosorbide plus importantes (et donc une teneur en matière biosourcée plus importante), ces compositions comprenant simultanément le monomère (A) et l'acrylate d'isosorbide comme monomère additionnel (B) sont plus brillantes que les compositions utiles à la présente invention exemptes d'un tel monomère (B).

## Revendications

1. Utilisation pour la fabrication de revêtement polymère d'une composition comprenant au moins un composé (A) de formule 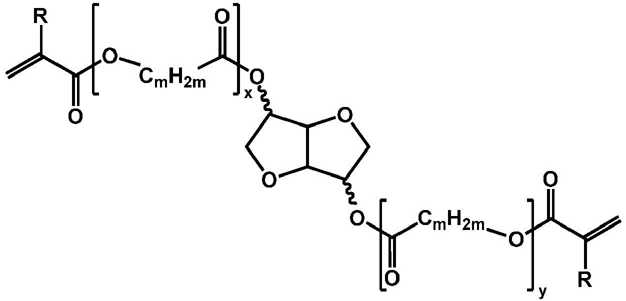 **caractérisé en ce que** les groupements R, identiques ou différents, sont choisis parmi les groupes H et CH₃ et :
• x est un entier allant de 0 à 100 ;
• y est un entier allant de 0 à 100 ;
• x+y est un entier supérieur ou égal à 1 ;
• m est un entier égal à 5.
un amorceur de polymérisation et au moins un monomère additionnel (B) susceptible de réagir avec ledit composé (A) et différent dudit composé (A).

2. Utilisation selon la revendication 1, **caractérisé en ce que** :
• x est un entier allant de 0 à 49 ;
• y est un entier allant de 0 à 49;
• x+y est un entier allant de 1 à 50.

3. Utilisation selon la revendication précédente, **caractérisé en ce que** :
• x est un entier allant de 0 à 14 ;
• y est un entier allant de 0 à 14;
• x+y est un entier allant de 2 à 15.

4. Utilisation selon l'une quelconque des revendications 1 à 3, **caractérisée en ce que** le monomère additionnel (B) est un (méth)acrylate de 1, 4 : 3, 6 dianhydrohexitol.

5. Utilisation selon l'une quelconque des revendications 1 à 3, **caractérisée en ce que** le monomère additionnel (B) est un monomère promoteur d'adhésion.

6. Utilisation selon la revendication 5, **caractérisée en ce que** le monomère promoteur d'adhésion comprend une fonction promotrice d'adhésion choisie parmi la fonction acide carboxylique, notamment les fonctions acrylique ou méthacrylique, les fonctions comprenant au moins un atome de phosphore sous forme acide ou ester, les fonctions éthoxy- ou méthoxy-silane ou les fonctions silanol.

7. Utilisation selon la revendication 6, **caractérisée en ce que** le monomère promoteur d'adhésion est choisi parmi l'acide acrylique, l'acide méthacrylique, les composés insaturés dérivés d'acide phosphorique et d'ester d'acide phosphorique, les composés insaturés dérivés d'acide phosphonique et d'ester d'acide phosphonique, les composés insaturés alcoxysilanes et les composés insaturés porteurs de la fonction silanol.

8. Procédé de fabrication d'un revêtement polymère sur un substrat comprenant les étapes suivantes :
- le dépôt sur le substrat d'une couche de la composition utilisée dans l'une quelconque des revendications 1 à 7, puis
- la réticulation de la composition.

9. Procédé selon la revendication précédente, **caractérisé en ce que** le substrat est choisi parmi les substrats en bois, en métal, en plastique, en verre ou en papier.

## Patentansprüche

1. Anwendung einer Zusammensetzung, welche wenigstens eine Verbindung (A) mit der Formel 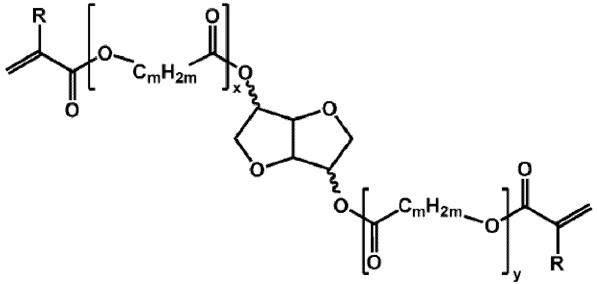
umfasst, zur Herstellung einer Polymerbeschichtung
**dadurch gekennzeichnet, dass** die identischen oder unterschiedlichen Gruppierungen R aus H- und CH₃-Gruppen gewählt sind, und:
* x eine ganze Zahl zwischen 0 und 100 ist;
* y eine ganze Zahl zwischen 0 und 100 ist;
* x+y eine ganze Zahl größer gleich 1 ist;
* m eine ganze Zahl gleich 5 ist,
einen Polymerisationsstarter und wenigstens ein zusätzliches Monomer (B), welches mit der Verbindung (A) reagieren kann und von der Verbindung (A) verschieden ist.

2. Anwendung nach Anspruch 1, **dadurch gekennzeichnet, dass**:
* x eine ganze Zahl zwischen 0 und 49 ist;
* y eine ganze Zahl zwischen 0 und 49 ist;
* x+y eine ganze Zahl zwischen 1 und 50 ist.

3. Anwendung nach dem vorhergehenden Anspruch, **dadurch gekennzeichnet, dass**:
* x eine ganze Zahl zwischen 0 und 14 ist;
* y eine ganze Zahl zwischen 0 und 14 ist;
* x+y eine ganze Zahl zwischen 2 und 15 ist.

4. Anwendung nach einem der Ansprüche 1 bis 3, **dadurch gekennzeichnet, dass** das zusätzliche Monomer (B) ein 1,4:3,6-dianhydrohexit-(meth)acrylat ist.

5. Anwendung nach einem der Ansprüche 1 bis 3, **dadurch gekennzeichnet, dass** das zusätzliche Monomer (B) ein Monomer-Haftvermittler ist.

6. Anwendung nach Anspruch 5, **dadurch gekennzeichnet, dass** der Monomer-Haftvermittler eine Haftvermittlerfunktion aufweist, welche gewählt ist aus Carboxyfunktion, insbesondere Acryl- oder Methacryl-Funktion, wobei die Funktionen wenigstens ein Phosphoratom in Säure- oder Esterform, Ethoxy- oder Methoxy-Silan oder Silanolfunktionen aufweisen.

7. Anwendung nach Anspruch 6, **dadurch gekennzeichnet, dass** der Monomer-Haftvermittler gewählt ist aus Acrylsäure, Methacrylsäure, ungesättigte Verbindungen als Derivate von Phosphorsäure und Phosphorsäureester, ungesättigte Verbindungen als Derivate von Phosphonsäure und Phosphonsäureester, ungesättigte Alxoxysilanverbindungen und ungesättigte Verbindungen als Träger der Silanolfunktion.

8. Verfahren zur Herstellung einer Polymerbeschichtung auf einem Substrat, welches die folgenden Schritte umfasst:
- Aufbringen einer Schicht der in einem der Ansprüche 1 bis 7 verwendeten Verbindung auf das Substrat, dann
- Vernetzung der Verbindung.

9. Verfahren nach dem vorhergehenden Anspruch, **dadurch gekennzeichnet, dass** das Substrat aus Holz-, Metall-, Kunststoff-, Glas- oder Papiersubstraten gewählt wird.

## Claims

1. Use for the production of polymer coatings of a composition comprising at least one compound (A) represented by the formula 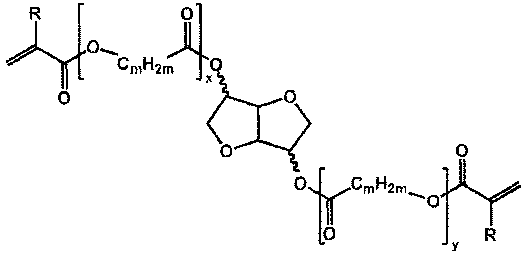 **characterised in that** the groups R, identical or different, are chosen among the groups Hand CH₃ and:
• x is an integer ranging from 0 to 100;
• y is an integer ranging from 0 to 100;
• x+y is an integer greater than or equal to 1;
• m is an integer equal to 5.
a polymerisation primer and at least one additional monomer (B) suitable for reacting with said compound (A) and different from said compound (A).

2. Use according to claim 1, **characterised in that**:
• x is an integer ranging from 0 to 49;
• y is an integer ranging from 0 to 49;
• x+y is an integer ranging from 1 to 50.

3. Use according to the preceding claim, **characterised in that**:
• x is an integer ranging from 0 to 14;
• y is an integer ranging from 0 to 14;
• x+y is an integer ranging from 2 to 15.

4. Use according to any one of claims 1 to 3, **characterised in that** the additional monomer (B) is a (meth)acrylate of 1,4:3,6 dianhydrohexitol.

5. Use according to any one of claims 1 to 3, **characterised in that** the additional monomer (B) is an adhesion promoter monomer.

6. Use according to claim 5, **characterised in that** the adhesion promoter monomer comprising an adhesion promoting function chosen among the carboxylic acid function, particularly the acrylic or methacrylic functions, functions comprising at least one phosphorus atom in acid or ester form, ethoxy- or methoxy-silane functions or silanol functions.

7. Use according to claim 6, **characterised in that** the adhesion promoter monomer is chosen among acrylic acid, methacrylic acid, unsaturated compounds derived from phosphoric acid and phosphoric acid ester, unsaturated compounds derived from phosphonic acid and phosphonic acid ester, unsaturated alkoxysilane compounds and unsaturated compounds carrying the silanol function.

8. Method for producing a polymer coating on a substrate comprising the following steps:
- deposition on the substrate of a layer of the composition used in any one of claims 1 to 7, then
- crosslinking of the composition.

9. Method according to the preceding claim, **characterised in that** the substrate is chosen among wood, metal, plastic, glass or paper substrates.
